# EUROPEAN PATENT APPLICATION

(11) **EP 4 847 481 A1**
(43) Date of publication of application: **02.12.2026**
(21) Application number: 25178848.5
(22) Date of filing: 26.05.2025
(51) Int. Cl.: C07C 41/01, C07C 43/23

(54) **PROCESS FOR REDUCTIVE CATALYTIC DEPOLYMERIZATION OF LIGNIN**

(71) Applicant: VITO NV, 2400 Mol (BE)
(72) Inventor: SRIDHARAN, Balaji, 2400 Mol (BE); DAELEMANS, Brent, 2400 Mol (BE); JUSNER, Paul, 2400 Mol (BE)
(74) Representative: Arnold & Siedsma

(57) **Abstract**

The process involves the reductive catalytic depolymerization of lignin, starting with premixing lignin with a depolymerized lignin in a specific weight ratio. This mixture is then liquified by adding an organic alcohol and heating it below the alcohol's boiling point. The liquified mixture undergoes depolymerization, resulting in a product mixture of organic alcohol and depolymerized lignin. The product mixture is separated into fractions, with part of one fraction recycled back into the process. The process can include pressurization and is performed at specific temperatures using a heterogeneous catalyst. Various types of lignin and organic alcohols can be used, and the process aims to minimize carbon losses.

## Description

### TECHNICAL FIELD

The present disclosure relates to a process for reductive catalytic depolymerization of lignin, and more particularly, to a method involving premixing lignin with depolymerized lignin, liquifying, depolymerizing, and recycling components to achieve efficient lignin breakdown.

### BACKGROUND

Lignin is a complex organic polymer found in the cell walls of plants, particularly in wood and bark, and is one of the most abundant renewable resources of aromatic compounds on Earth. It provides structural support and protection to plants, making it a crucial component of biomass. However, lignin is often considered a waste product in the paper and biofuel industries due to its complex and recalcitrant nature, which makes it difficult to break down and convert into valuable products. Traditional methods of lignin depolymerization often result in significant carbon losses and produce char, which can lead to operational challenges such as clogging and blockages in processing equipment.

Lignin depolymerization to valuable biochemicals and bioaromatics has been widely researched as a sustainable approach to produce renewable fuels and chemicals. Key strategies reported include thermal, catalytic, and solvent-assisted depolymerization methods. However, few viable approaches have been demonstrated in a continuous scale to efficiently depolymerize lignin at the necessary scale.

Some methods have been reported for converting lignin into more valuable materials. However, these processes are not as advanced as those for carbohydrate conversion. Amongst the methods reported, reductive catalytic depolymerization of lignin appears to be an efficient and scalable way to produce valuable bioaromatic molecules from lignin. However, typical yields from process reported are often low: due to low solubility of lignin in traditional Reductive Catalytic Depolymerization (RCD) solvents (low boiling alcohols) and losses due to formation of char. Lan et al. demonstrated the continuous depolymerization of acetal stabilized lignin using a solvent mixture of dioxane and methanol (Lan et al., 2021). Oil yields of about 80 wt% are reported with a decline in catalytic activity measured after 10 H of operation. Jusner et al. demonstrated the continuous reductive catalytic depolymerization of hydrolysis lignin with a stable operational time of over 80H (Jusner et al., 2024). However, limited solubility of lignin (approximately 50 - 60 wt%) of lignin in the solvent used (methanol) caused a large loss in overall carbon and product yields. Other organic solvents such as γ-Valerolactone have been suggested as more greener and biobased alternatives to more toxic polar solvents such as dioxane for lignin depolymerization (Cheng et al., 2023; Jasiukaityte-Grojzdek et al., 2020). Solubility of lignin in such solvents is not well explored and thus limiting such research to lab scale experiments in pressured batch reactors. Sridharan et al. demonstrated the use of monomeric lignin derived phenols such as cresol for dissolving and feeding lignin into a pressured autoclave reactor using a piston pump for depolymerization using high temperature hydropyrolysis (temperature > 300 °C) (Sridharan et al., 2024). Complete solubility of lignin enabled the continuous operation of such depolymerization process. However, the high boiling point of the monomeric phenols used (boiling temperature > 210 °C) made removal and recycle expensive, tedious and more importantly poses an additional cost on such a process and has a direct impact on its overall technoeconomic feasibility.

The need for efficient and sustainable methods to depolymerize lignin has become increasingly important as industries seek to utilize biomass more effectively and reduce waste. Reductive catalytic depolymerization is a promising approach that involves breaking down lignin into smaller, more useful molecules using catalysts and reducing agents. This process has the potential to convert lignin into valuable chemicals and fuels, thereby enhancing the economic viability of biomass utilization. However, challenges such as solvent loss, carbon retention, and continuous operation without equipment fouling remain significant hurdles that need to be addressed to make lignin depolymerization a commercially viable process.

### SUMMARY

As mentioned herein before, one of the major problems related to development of a continuous process for the depolymerization of lignin on an industrial scale is the limited ability of the traditional hydrogenolysis solvents to dissolve lignin, and thus making handling and pumping (pressurizing) of such a slurry feed mixture complicated and impractical. Other solvents such as dioxane and acetone can dissolve lignin well but are often reactive during the depolymerization process and thus lead to excess solvent consumption and possibly contamination of the lignin derived depolymerization products.

Instead of using the traditional low boiling alcohols as hydrogenolysis solvents, higher boiling solvents such as dimethyl sulfoxide (DMSO) and lignin derived phenols (cresol) can be used instead and are shown to dissolve lignin well but recycling them often requires an expensive distillation step which may also degrade the quality of the lignin depolymerization products, especially from a process such as reductive catalytic depolymerization where the temperature of depolymerization used( 200 - 250°C) roughly corresponds to the boiling point of the phenolic monomer used.

It is an objective of the present disclosure in providing a method that addresses the aforementioned problems and enables an improved method for the reductive catalytic depolymerization of lignin, comprising the steps of; a) premixing a lignin (L) to be depolymerized with a (first) (preferably liquid) depolymerized lignin (DL) in a weight ratio of DL/L of at least 0.5, thereby obtaining a Premixed Lignin (PL); b) liquifying the PL by adding an organic alcohol and heating with optionally pressurizing the thus obtained mixture at a temperature and pressure such that the organic alcohol is liquid, for a time sufficient to liquify the PL, thereby obtaining a liquified PL; c) depolymerizing the liquified PL using reductive catalytic depolymerization, thereby obtaining a Product Mixture (PM) of the organic alcohol and a depolymerized lignin; and d) removing at least 90% (wt) of the organic alcohol from the PM, thereby obtaining a (second) (preferably liquid) depolymerized lignin (DL2).

The process, in particular from steps b) till step d), can be conducted under pressure to prevent solvent loss and may be continuous to enhance efficiency. The method may minimize carbon losses, ensuring a yield of depolymerized lignin, which can be advantageous for both economic and technical reasons.

The (second) (preferably liquid) depolymerized lignin (DL2) obtained in step d) and the (first) (preferably liquid) depolymerized lignin (DL1) used in step a) can be the same or different.

In the premixing step the weight ratio of DL/L typically ranges from 0.5 to 5; in particular from 0.5 to 2. Within said ratios the subsequent liquification with a low-boiling organic alcohol (S) was found to result in a significant increase in the solubility of the lignin in the liquified premixed lignin, with for the majority of the tested lignins a complete dissolution. In liquifying the PL, the mixing conditions regarding temperature and pressure are set to have the organic alcohol in a liquid state. The liquification of the premixed lignin (PL) will beneficially be performed at an increased temperature but below the boiling temperature of the organic alcohol used, i.e. at temperatures and pressures at which the organic alcohol is in liquid state. In an embodiment the premixing is performed at temperatures up to 150°C; in particular at temperatures ranging from 50°C to 150°C, with pressures up to 100 bar; in an embodiment ranging from above 1 to 25 bar; in another embodiment from 10 to 15 bar; alternatively from 10 to 30 bar. In a particular embodiment ranging from 10 to 100 bar. Pressurizing during the premixing, using for example an extruder, can facilitate the subsequent liquification of the Premixed Lignin (PL) using the organic alcohol.

In principle any organic alcohol with a boiling point up to 150°C at atmospheric pressure can be used as a solvent (S) in the liquification of the PL. The traditional low boiling alcohols known for their application as hydrogenolysis solvents being preferred, such as for example selected from the group consisting of methanol, ethanol, propanol, butanol, and mixtures thereof.

Per reference to the examples hereinafter, in as far the liquification in accordance with the herein disclosed method results in a significant, and for the majority of lignins a complete dissolution, solid materials could still be present in the liquified PL. Thus, in a particular embodiment the process further comprises a separation step wherein solids are removed from the liquified PL prior to the depolymerizing step c); in particular using a filtration system.

The liquified PL will then undergo a depolymerization process using reductive catalytic depolymerization. The depolymerization is typically performed at elevated temperatures ranging from 200°C to 270°C, and may involve the use of a heterogeneous catalyst. The use of such a catalyst can aid in the effective depolymerization of the liquified PL, resulting in a Product Mixture (PM) that comprises the organic alcohol (S) and depolymerized lignin (DL). In an embodiment the heterogenous catalyst is typically composed of noble metals such as Pd/Ru/Pt/Rh and the like or transition metal catalysts such as Ni/Co/Cu/Mo.

Following the depolymerization, the Product Mixture (PM) may be separated into at least two fractions with the separation of the organic alcohol (S) from the depolymerized lignin (DL) present in the Product Mixture (PM). This separation can for example be achieved through the evaporation of the organic alcohol mixture, which may facilitate the recycling of a part of a (preferably liquid) fraction of depolymerized lignin back to the initial premixing step. The recycling of this fraction may enhance the efficiency of the process and ensure the continuous operation of the system without any clogging or blockage issues. Hence in a preferred embodiment the process further comprises a step e) of recycling a fraction of the (preferably liquid) depolymerized lignin obtained in step d) to step a).

In an embodiment the separated organic alcohol is recycled to the liquification of the PL. In said embodiment the process further comprises a step f) of recycling the separated organic alcohol obtained in step d) to step b).

The process may also include the step of pressurizing the liquified PL to a pressure up to 100 bar; in an embodiment ranging from above 1 to 25 bar; in another embodiment from 10 to 15 bar; alternatively from 10 to 30 bar. In a particular embodiment ranging from 10 to 100 bar. This pressurization can prevent the loss of the organic solvent upon exposure to the reductive catalytic depolymerization. The pressurization step may be particularly important when the depolymerization is performed temperatures above the boiling point of the organic alcohol used in the liquification step, as it can help maintain the integrity of the organic solvent within the system.

Pre-pressurizing the liquified PL prior to the depolymerization step also allows coordination of the liquification with the depolymerization step as the latter is preferably performed not only at such higher temperatures, but also at pressures ranging from 10 to 100 bar; in particular from 30 to 90 bar; more in particular from 40 to 80 bar. The pressured and liquified PL can be fed directly into depolymerization process. To said extent and as a provided in the examples hereinafter, in an embodiment the premixing of step a) is done using a screw extruder system. Such screw extruder enables the handling and pumping (pressurizing) of the slurry feed of Premixed Lignin (PL) initially obtained, to match the pressure conditions used during the depolymerization step. As such, it reduces the need in further equipment in preparing the pressurized and liquified PL.

Overall, the process may aim to minimize carbon losses as undissolved lignin during dissolution and as char during depolymerization. This minimization of carbon losses can be beneficial from both an economic and technical perspective, as it may allow for continuous operation without any interruptions due to clogging or blockage by char or undissolved lignin. The process may be designed to be a continuous process, ensuring the efficient and effective depolymerization of lignin into valuable products.

In the process, the organic alcohol may be removed from the PM, which may facilitate the separation of the PM into at least two fractions with the separation of the organic alcohol (S) from the depolymerized lignin (DL) present in the Product Mixture (PM). As will be appreciated by the skilled artisan, some residual organic alcohol can remain in the DL fraction. Within the context of the present invention, removal of the organic alcohol implies that at least 90% (wt) of the organic alcohol is removed from the DL; in particular at least 91, 92, 93, 94, 95, 96, 97, 98 or 99% (wt). The removal of the organic alcohol, can be achieved through evaporation or other separation techniques, such as for example a flash step or by distillation under reduced pressure. The separated organic alcohol obtained from this process may be recycled back into the liquification step, thereby creating a continuous loop that can enhance the efficiency and sustainability of the process. The recycling of the organic alcohol may contribute to minimizing waste and optimizing the use of resources within the system. The entire process may be designed to operate continuously, allowing for a steady production of depolymerized lignin with minimal interruptions.

Upon removal of the organic alcohol, the depolymerized lignin obtained from the Product Mixture (PM) may be separated into fractions, which can allow for the recycling of a part of a fraction of depolymerized lignin (DL) back to the initial step. The separation of the Product Mixture (PM) into at least two identical (preferably liquid) fractions of DL may be achieved, which can be beneficial for maintaining the continuity of the process. The recycling of the depolymerized lignin (DL) may contribute to the efficiency of the process by ensuring that a portion of the initial lignin is reused, potentially reducing waste and improving the overall yield. The evaporation of the organic alcohol may also play a role in the separation process, as it can help in isolating the depolymerized lignin (DL) from the mixture. This step of removing the alcohol may contribute in the effective recycling of the depolymerized lignin, as it can ensure that the depolymerized lignin is in a suitable form for reuse in the initial premixing step. The process may be designed to accommodate the recycling of the depolymerized lignin, which can be a factor in achieving a sustainable and efficient depolymerization process.

In the process, lignin may be selected from a group to ensure its suitability for the depolymerization process as herein provided and characterized in having a low solubility in the traditional low boiling alcohols as hydrogenolysis solvents. Per reference to the examples hereinafter, a low solubility corresponds to a solubility of less than 70 wt%, calculated based on the initial lignin mass, in methanol at a temperature of 120°C and a pressure of 10 bar. The lignin materials are derived from lignocellulosic biomass and include technical lignins such as kraft lignin, soda lignin, hydrolysis lignin, organosolv lignin, lignins derived from pyrolysis of biomass, and lignin obtained from the reductive fractionization and delignification of biomass.

Depolymerized lignin (DL) as used herein, generically refers to lignin that has undergone a chemical or enzymatic process to break its polymer structure into smaller molecular components. Lignin is a complex polymer found in the cell walls of plants, and it provides rigidity and protection. In the past few decades, various lignin depolymerization strategies have been developed, which can be generally categorized as: reductive, oxidative, acid-catalyzed, base-catalyzed, thermal methods, etc. (Sun et al. 2018, Behling et al. 2016, Li et al. 2015, Schutyser and Kruger et al. 2018). In general, a depolymerization process breaks the interunit linkages within the lignin macromolecule, converting complex lignin polymers into oligomers or monomeric aromatic products that can be further modified for various applications.

In a particular embodiment the depolymerized lignin, hereinafter also referred to as DL, used in the methods of depolymerization as herein provided can be characterized in having a number average molecular weight (Mn) ranging from 200 to 1500 g/mol, a weight average molecular weight (Mw) ranging from 200 to 4000 g/mol, and a polydispersity index ranging from 1 to 4.

### BRIEF DESCRIPTION OF THE DRAWINGS

Figure 1: Process overview for continuous and efficient depolymerization of lignin and the individual product yields from each of the process steps involved.
Figure 2: Solubility (wt %) of different lignins in the chosen solvent systems
Figure 3: Yield of Depolymerized Lignin (DL) and the solubility of the lignin during different recycle passes
Figure 4: Relative concentration of different alkylphenol monomers in the DL
Figure 5: Effect of product (DL) recycle on its average molecular weight and polydispersity index (PI)
Figure 6: OH-content of the lignin and Depolymerized Lignin (DL) measured from the recycle experiments.
Figure 7: Process pressure measured over the reactor bed during the first pass of recycle.
Figure 8: Process pressure measured over the reactor bed during the second pass of recycle.

### DETAILED DESCRIPTION

The present invention relates to a process for reductive catalytic depolymerization of lignin comprising the steps of:
a) Premixing a lignin (L) to be depolymerized with a (first) (in particular liquid) depolymerized lignin (DL1) in a weight ratio of DL1/L of at least 0,5 thereby obtaining a Premixed Lignin (PL)
b) Liquifying the PL by adding an organic alcohol and heating with optionally pressurizing the thus obtained mixture at a temperature and pressure at which the organic alcohol is liquid, for a time sufficient to liquify the PL, thereby obtaining a liquified PL;
c) Depolymerizing the liquified PL using reductive catalytic depolymerization, thereby obtaining a Product Mixture (PM) (in particular in a liquid state) of the organic alcohol and depolymerized lignin, and
d) Removing the organic alcohol from the PM, thereby obtaining a (second) (in particular liquid) depolymerized lignin (DL2).

The (in particular liquid) depolymerized lignin (DL2) obtained in step d) can be split in two fractions wherein at least a part of a fraction of depolymerized lignin (DL2-F2) from step d) is recycled to step a).

As the reductive depolymerization is typically performed at temperatures ranging from 200 - 270°C , the liquified PL is preferably pressurized under said conditions, in order to prevent loss of the organic solvent upon exposure to the reductive catalytic depolymerization. In an embodiment the process includes a step of pressurizing the liquified PL to a pressure up to 100 bar (supra) prior to the depolymerization, thereby obtaining a pressurized liquified PL.

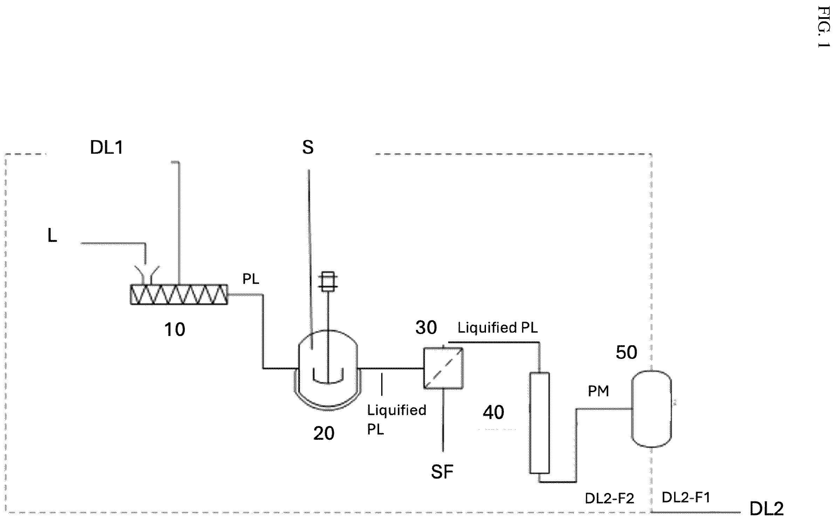

In an embodiment the pressurizing can already be part of the premixing of the lignin with the depolymerized lignin, for example when making use of a screw extruder (10), in said instance the (first) depolymerized lignin can be fed into the screw extruder loaded with the lignin to be depolymerized and pressurized to the aforementioned pressures when fed into a dissolution tank (20) (see figure 1). In an alternative configuration both the (first) (in particular liquid) depolymerized lignin and the organic alcohol are fed into the screw extruder loaded with the lignin to be depolymerized and pressurized to the aforementioned pressures when fed into a dissolution tank.

The screw extruder as used herein includes single-screw extruders and twin-screw extruders. Material is fed into a barrel comprising one or two screw(s) that convey the material forward, applying pressure and often heat, forcing the material through a die at the end of the barrel. The barrel can be heated, in the present instance to accelerate the liquification of the lignin to be depolymerized. In an embodiment step a) is performed using a screw extruder, in particular a screw extruder heated up to and below the boiling point at atmospheric pressure of the organic alcohol used in the liquification step. More in particular at temperatures up to 150°C; in particular at temperatures ranging from 50°C to 150°C.

The lignin (L) used in the invention may be any type of lignin and is provided in particulate form with a particle size ranging from 150 nm to 2 mm. Said lignin materials are derived from lignocellulosic biomass by techniques such as chemical pumping (soda, sulfite, acid and kraft), biomass delignification (organosolv or hydrolysis) and fractionization. The particle size distribution of lignin is known to vary depending on the extraction process used, for example organosolv lignin can have a wide range of particle sizes, from less than 0.25 mm to 2.0 mm, whereas Kraft lignin typically exhibits a bimodal particle size distribution, with peaks around 605.5 nm and 475.8 nm, and lignosulfonates obtained from the sulfite process generally have a particle size distribution ranging from 8 to 20 micrometers.

The lignins as used herein, may be selected from a group to ensure its suitability for the depolymerization process as herein provided and characterized in having a low solubility in the traditional low boiling alcohols as hydrogenolysis solvents. Per reference to the examples hereinafter, a low solubility corresponds to a solubility of less than 70 wt%, calculated based on the initial lignin mass, in methanol at a temperature of 120°C and a pressure of 10 bar. Such lignins typically have a carbon content from 45 - 80 mol%, a hydrogen content of 5 - 10 mol% and an oxygen content of 10 - 50 mol%. For example, lignins may have a C/H/O ratio ranging between 45/5/50 to 80/10/10. Specific examples of lignins are technical lignins such as kraft lignin, soda lignin, organosolv lignin, lignins derived from pyrolysis of biomass, and lignin obtained from the reductive fractionization and delignification of biomass.

In the premixing step the depolymerized lignin (DL) generally refers to a biopolymer mixture obtained from the depolymerization of a lignin and composed of repeating (oligomers) and non-repeating (monomers) phenolic molecules. The number average molecular weight (Mn) may range from 200 to 1500, while the weight average molecular weight (Mw) may range from 200 to 4000, and the polydispersity index (PDI), being the ratio of Mw over Mn may range from 1 to 4. The molar mass parameters MW and Mn can be determined using procedures known in the state of the art, such as the Gel Permeation Chromatography (GPC) protocol disclosed in Jusner P. et al, Chemical Engineering Journal 499 (2024), 156474.

In addition to the aforementioned molecular mass parameters, the depolymerized lignin (DL) is preferably in liquid form, and could further be characterized based on the OH-distribution as determined by P31 NMR according to Gracia-Vitoria et al., Ind. Crop. Prod. 176 (2022) 114405. Compared to polymeric lignin, in depolymerized lignin the content of aliphatic OH-functionalities (AlOH) is reduced and the content in condensed aromatic OH-moieties (CarOH) and uncondensed aromatic OH-moieties (UArOH) increased. For the depolymerized lignin the content of AlOH expressed in mmol/g ranges from 2,0 to 5,5; the content of CarOH expressed in mmol/g ranges from 2,0 to 3,1; and the content of UrOH expressed in mmol/g ranges from 0,75 to 4,00.

Hence in a particular embodiment according to the invention, the (in particular liquid) depolymerized lignin (DL) used in the process according to the invention, consists of a (preferably liquid) depolymerized lignin characterized by means of the molecular mass parameters and the OH distribution as specified in the above mentioned paragraphs; in particular with for the molecular mass parameters Mn ranging from 200 to 1500, Mw ranging from 200 to 4000, and PDI ranging from 1 to 4; and the OH distribution expressed as the relative content in mol/g for AlOH ranging from 2,0 to 5,5; for CarOH from 2,0 to 3,1 and for UrOH ranging from 0,75 to 4,00.

As already mentioned hereinbefore, the organic alcohol solvent (S) used in the liquification of the Premixed Lignin (PL) can be any compound with a molecular structure CₙH₂ₙ₊₁OH with a maximum boiling point of 150 °C at atmospheric pressure. Typical examples include: methanol, ethanol, propanol, butanol etc.

It has been found that the liquification with such low boiling alcohols, of the lignins as herein provided, is made possible by premixing (step a)) said lignins with a (preferably liquid) depolymerized lignin, wherein the weight ratio of DL/L is at least 0.5 and preferably ranges from 0.5 to 5; more in particular from 0.5 to 3; even more in particular from 0.5 to 2. Under said conditions a completely miscible mixture with little to no solid precipitates is obtained under mild conditions, i.e. at temperatures up to 150°C; in particular at temperatures ranging from 50°C to 150°C, already after a short exposure to the organic alcohol. This enables a continuous process wherein the Premixed Lignin (PL) and organic alcohol are fed into a dissolution reactor (20) and passed on to the depolymerization step as liquified PL after a residence time as short as 5 minutes. Evidently, the step can also be performed in a batch mode, wherein the residence time ranges from 5 minutes to 1 hour.

Optionally the liquified PL may undergo a separation step wherein solids (SF) are removed from the liquified PL prior to the depolymerization step. In principle any known separation system to remove solids from a liquid (30) can be used, such as filtration or centrifugation, filtration being preferred.

The reductive catalytic depolymerization is typically performed at elevated temperatures ranging from 200°C to 270°C, at pressures ranging from 10 to 100 bars with a hydrogen source present and may involve the use of a heterogeneous catalyst. The depolymerization can be performed in a continuous mode using a packed bed reactor (40) or in batch mode using a pressured autoclave reactor. The liquefied lignin mixture undergoes depolymerization leading to reduction in its average molecular weight. The final product is a product mixture (PM), typically in a liquid state, of the organic alcohol and depolymerization products of the lignin, consisting of a combination of monomers, dimers, trimers and heavier oligomers composed of alkylphenol molecules.

To obtain the (second) depolymerized lignin (DL2), the organic alcohol still needs to be removed from said (in particular liquid) mixture. Art known solvent removal techniques (50) can be used and are not restricted to evaporation (such as flash evaporation, or rotary evaporation), pervaporation (membrane separation) or distillation (such as fractional distillation, steam distillation, vacuum distillation, and the like); flash evaporation and distillation being preferred.

In a particular embodiment of the present invention, schematically shown in Figure 1, the process for reductive catalytic depolymerization of lignin, can be summarized by the following steps;

### Step a: Mild temperature dissolution

Lignin (L), depolymerized lignin (DL1) preferably as a fraction (DL2-F2) of the depolymerized lignin (DL2) obtained from step d along with the organic alcohol, ie. methanol (S) is mixed together at mild temperatures (<150 °C) to obtain a completely miscible mixture with little to no solid precipitates. Additionally, mixture of the lignin and the depolymerized lignin makes a very viscous paste which can be fed to a dissolution reactor using a screw extruder system (10) operating at very mild temperatures. Organic alcohol (S) is then added to the dissolution reactor using a dosing pump, with a flow rate dependent on the mass of lignin used. After a residence time of at least 5 minutes, the liquid mixture is passed through a particulate filter system (30) to separate any undissolved solids (if any).

### Step b: Reductive catalytic depolymerization of liquified lignin mixture:

The liquefied lignin mixture (liquified PL) is depolymerized at temperatures around 200 - 270 °C at an operating pressure of 10 - 100 bars with a hydrogen source present, either molecular hydrogen or via a hydrogen transfer agent. Reductive heterogenous catalysts composed of noble metals such as Pd/Ru/Pt/Rh etc or transition metal catalysts such as Ni/Co/Cu is used to catalyze this reaction. Reductive catalytic depolymerization can either be performed in a continuous mode using a packed bed reactor or in batch mode using a pressured autoclave reactor (40). The liquified lignin mixture undergoes depolymerization leading to reduction in its average molecular weight. The final product is a Product Mixture (PM) of the organic alcohol and of lignin derived monomers, dimers, trimers and heavier oligomers composed of alkylphenol molecules.

### Step c: Evaporation of organic alcohol mixture:

The organic alcohol solvent (S) used can removed from the depolymerized lignin oil through a flash step or by distillation under reduced pressure (50). Flashing involving temperatures up to 150 °C (temperature of step b) and pressure of 0.01 bar to 1.5 bar is used to separate the alcohol solvent from the Product Mixture (PM) comprising the Depolymerized lignin (DL2).

### Step d: Separation and recycle of part of the depolymerized lignin to step a:

Part of the solvent-free (organic alcohol) depolymerized lignin (DL2-F2) (in particular in liquid state) is recycled back to step a to increase the solubility of the lignin used. The part of the depolymerized lignin (DL2-F1) not recycled is then used in subsequent biopolymer synthesis applications such as PU, epoxies and additives for flame retardants as demonstrated in the examples shown below.

### EXAMPLES

This and other aspects of the present invention will become apparent from the examples hereinafter.

### Methods:

### Chemicals:

Organic solvents such as methanol were purchased from VWR and were of technical grade purity (98%). Several lignins were used, originating from organosolv and hydrolysis-based extraction methods. Lignins were sourced directly from different commercial lignin suppliers.
.Heterogenous catalysts such as Palladium and Ruthenium on different supports such as Al₂O₃ and activated carbon support were purchased from Heraeus Precious Metals GmbH & Co. KG, Germany. Hydrogen gas was of technical grade and supplied by Air Products.

### Process Description:

### Solubilization of lignin in Batch

Lignin was dissolved in feed mixture of (first) Depolymerized Lignin (DL1) and methanol (MeOH) at mild temperatures between 25 °C and 120°C. The lignin, DL1 and methanol mixture in the mass ratio 20:20:60 was added to a stirred vessel at ambient temperature and pressure. The stirred vessel was sealed and pressurized to 10 bar using N2 as carrier gas. The pressurized vessel was then heated to the desired temperature (25 - 120°C) for 60 minutes and then the vessel was then allowed to cool down. The vessel when back at ambient temperature was depressurized and contents inside were recovered. Mass of the vessel before and after dissolution was measured to determine the mass loss due to gas formation (if any). The obtained feed mixture was passed through a filter (cellulose) to remove any undissolved solids. The filtrate was then washed with excess methanol and then dried in a vacuum oven at 45 °C overnight. The mass of dry insoluble filtrate was measured. The insoluble fraction of the lignin accounted for approximately 0 to 30 wt% (calculated based on the initial lignin mass) depending upon the type of lignin used. The organosolv-based lignins showed complete solubility while hydrolysis-based lignins showed relatively limited solubility (70 wt% approx.) in the solvent mixture used.

### Continuous solubilization of lignin using a screw extruder.

Hydrolysis lignin and (first) liquid depolymerized lignin (DL1) were physically blended in a predetermined mass ratio, typically 1:1 (g/g), and introduced into a corotating twin-screw extruder (Three-tec ZE 12 HMI with an L/D ratio of 40). This extruder was equipped with six independently heated zones, enabling precise temperature control along the barrel length to optimize thermal processing. The screw configuration incorporated four distinct types of elements-conveying, kneading, backmixing, and reverse-designed to promote effective mixing, dispersion, and homogenization of the lignin-DL1 feedstock. When the mixture was heated above the glass transition temperature (Tg) of DL1 (typically in the range of 50-75 °C), it transitioned into a viscous, homogeneous paste. The processed material exhibited sufficient mechanical integrity and rheological properties to be pressurized to at least 150 bar at a screw rotational speed of 60 rpm. Both the extruder barrel temperatures and screw rotational speed (rpm) were key parameters for controlling the pressure development within the system. The liquified lignin-DL1 mixture was subsequently transferred to a continuous, pressurized dissolution reactor. In this stage, an organic alcohol was introduced via a dosing pump, with the flow rate proportionally adjusted to match the mass throughput of lignin entering the extruder. Following a minimum residence time of five minutes, the resulting liquid stream was directed through a particulate filtration system (cellulose) to remove any undissolved solids. Notably, the incorporation of DL1 into the feed significantly influenced the solubilization behaviour of the lignin, with observations indicating a increased overall solubility in the presence of DL1, and use of a screw extruder in preparing the premixed lignin (PL) enables a continuous lignin depolymerization process.

### Reductive catalytic depolymerization (RCD) of lignin

Reductive depolymerization of lignin was performed both in batch mode and in continuous mode. Batch mode RCD was performed in a pressured autoclave reactor while continuous RCD was performed in a packed bed reactor filled with an active catalyst of choice.

### Reductive catalytic depolymerization (RCD) of lignin in Batch:

The reductive catalytic depolymerization of lignin was carried out in a 100 mL stainless steel batch reactor (Premex Reactors). The liquified Premixed Lignin (PL) mixture, a combination of lignin, depolymerized lignin oil and methanol was used as feedstock for depolymerization. In a typical reaction, a feed mixture composed of 5g of lignin, 5 g of (first) depolymerized lignin (DL1)and 15 g of methanol combined with a specific amount of Palladium catalyst (10 wt%, relative the mass of lignin added) was loaded into the reaction vessel. The reactor was then sealed and purged three times with N₂ (10 bar) to ensure the removal of air. Subsequently, the reactor was pressurized with 30 bar of H₂ under ambient conditions. The reactor was then heated to a temperature of 235 - 270 °C (15 °C/min) under a constant stirring of 700 rpm. After reaching the reaction temperature, the temperature was kept constant for the desired reaction time (120 minutes). After the reaction, the reactor was cooled to room temperature using flowing water. The reactor was then depressurized at ambient temperature. Mass of the reactor was measured before and after reaction to compute the mass of gas produced during depolymerization. Reactor content was collected by washing the reactor with methanol.

The obtained Product Mixture (PM) was separated by filtration using a glass filter crucible to separate the solid residue (catalyst + unconverted/residual lignin + char) from the liquid product. The solid residue was washed seral times with acetone and dried at 80 °C for 24 h followed by equilibration with air humidity. The liquid product was rotatory evaporated to yield dark brown (second) Depolymerized Lignin (DL2). The DL2 was dried at 45 °C under vacuum for 12h and weighed to determine the yield of DL2. Mass of the solids obtained after drying was measured to determine the yield of char. The following definitions were used to calculate the respective product yields: $\begin{matrix}Depolymerized Lignin \left(DL\right) Yield \left(wt%\right) = \\ \frac{\left(Weight of dried product \left(g\right)-Weight of depolymerized lignin used in feed \left(g\right)\right)}{Weight of Initial Dried Lignin in Feed \left(g\right)} ∗ 100\end{matrix}$ $Monomer Yield \left(wt%\right) = \frac{Weight of phenolic monomers \left(g\right)}{Weight of dried DL \left(g\right)} ∗ 100$ $Char yield \left(wt%\right) = \frac{Weight of residual fraction \left(g\right) - Weight of catalyst \left(g\right)}{Weight of initial dried lignin in feed \left(g\right)} ∗ 100$ $Gas Yield \left(wt%\right) = \frac{Δ Weight of reaction vessel before and after reaction \left(g\right)}{Weight of initial dried lignin in feed \left(g\right)} ∗ 100$

### Continuous Reductive catalytic depolymerization (RCD) of lignin:

The reductive depolymerization of lignin was done in a custom-built continuous packed bed reactor. The system comprised a heated feed vessel (the temperature of the Liquified Premixed Lignin (PL) was set to 40 °C) equipped with an overhead stirrer, a HPLC pump (the feed flow rate was set to 25 mL/h), a heat traced feed line (the temperature was set to 50 °C) leading to the packed bed reactor filled with catalyst of choice placed in a vertically split reactor furnace, and a product collection vessel. The H₂ flow was set to 1500 - 2500 NmL/h by means of a mass flow controller and the reaction pressure was set to 80 bar by means of a back pressure regulator. All tubing and valves were obtained from Swagelok Company, USA. The cylindrical packed bed reactor had a diameter of 1/2". The reactor was packed with the desired amount (i.e., 7.5 g - 35 g) of catalyst. The catalyst was kept in position by filling the top and bottom of the reactor with glass beads. The catalyst granules and the glass beads were separated from each other with a layer of glass wool. The collected Depolymerized Lignin (DL) was pre-dried by means of rotary evaporation and was further dried until constant weight in a vacuum oven at 45 °C. Product yields from the depolymerization reaction were computed using the following definitions: $\begin{matrix}Depolymerized Lignin \left(DL\right) Yield \left(wt%\right) = \\ \frac{\left(Weight of dried product \left(g\right)-Weight of depolymerized lignin used in feed \left(g\right)\right)}{Weight of Initial Dried Lignin in Feed \left(g\right)} ∗ 100\end{matrix}$ $Monomer Yield \left(wt%\right) = \frac{Weight of phenolic monomers \left(g\right)}{Weight of dried DL \left(g\right)} ∗ 100$ $Char yield \left(wt%\right) = \frac{Weight of residual fraction \left(g\right) - Weight of catalyst \left(g\right)}{Weight of initial dried lignin in feed \left(g\right)} ∗ 100$

### Analytical Methods:

**Gel permeation chromatography (GPC): 1. Alkaline GPC** - For analysis, 10 mg of the sample material was dissolved in 3.3 mL of aqueous 0.1 M NaOH at RT. The GPC system comprised a Shimadzu Prominence autosampler, degasser, pump, column oven, refractive index detector, and UV detector (Shimadzu Europa GmbH, Duisburg, Germany). The columns used were a Ultrahydrogel 500 column (300 mm × 7.8 mm, 10 µm particle size) and a Ultrahydrogel 120 column (300 mm × 7.8 mm, 6 µm particle size) in series covering a separation range from 100 g/mol to 400000 g/mol (Waters, Etten-Leur, The Netherlands). A 100 µL sample aliquot was injected by the autosampler. The eluent was a solution of 0.1 M NaNO₃ (aqueous) and acetonitrile 80/20 (v/v). The pH of the eluent was adjusted to 11 by adding drops of a 50 wt% aqueous NaOH solution. The column temperature was 40 °C, the flow rate was 0.4 mL/min, and the molar mass parameters *M_{w}* and *Mₙ* were calculated based on a calibration with dextrane standards (covering a range of 180 g/mol to 401300 g/mol).

**2. THF GPC** - For analysis, 10 mg of the sample material was dissolved in 1 mL THF at RT. For THF GPC the same liquid chromatography instrumentation was used but with different columns and eluent. The columns used were a Styragel HR 0.5 column (300 mm x 7.8 mm, 5 µm particle size) and a Styragel HR 1 column (300 mm x 7.8 mm, 5 µm particle size) in series covering a range of 0 g/mol up to 100000 g/mol (Waters, Etten-Leur, The Netherlands). A 100 µL sample aliquot was injected by the autosampler. THF was used as eluent, the column temperature was 40 °C, the flow rate was 0.8 mL/min, and Mw and Mn were calculated based on calibration with polystyrene standards (covering a range of 162 g/mol to 12980 g/mol).

**³¹P nuclear magnetic resonance spectroscopy (NMR):** ³¹P NMR was done according to Gracia-Vitoria et al. (2022).[1] All preparational steps were done in a glove box under Ar atmosphere. First, the stock solution containing 269.57 mg of the internal standard (IS) N-hydroxy-5-norbornene-2,3-dicarboxylic acid imide (NHND, CAS: 21715-90-2, purity = 97%, Sigma-Aldrich), 24 mg of chromium acetylacetonate (Cr(acac)₃, CAS: 21679-31-2, purity = 97%, Sigma-Aldrich), 8 mL anhydrous deuterated chloroform (CDCl₃, CAS: 865-49-6, purity = 99.8 atom% D, Sigma-Aldrich), and 12.8 mL of anhydrous pyridine (CAS: 110-86-1, purity = 99.8%, Sigma-Aldrich) was prepared. Approximately 30 mg of sample material was transferred into a 1.4 mL vial and 0.75 mL of the stock solution was added. The vials were closed, and the samples were vortexed for 60 s. 0.1 mL of 2-chloro-4,4,5,5-tetramethyl-1,2,3-dioxaphospholane (TMDP, CAS: 14812-59-0, purity = 95%, Sigma-Aldrich) was added and the samples were vortexed for 60 s. Then, the samples were transferred into NMR tubes and placed into the autosampler. The instrument used was a benchtop Spinsolve 80 MHz Ultra series NMR (Magritek, Aachen, Germany) with a dual probe for ³¹P detection. The acquisition parameters were 512 scans, repetition time of 10 s, a pulse angle of 90°, the bandwidth was set to 154 ppm, the center frequency was set to 140 ppm, NOE enhancement and ¹H decoupling was applied.

**2D ¹H-¹³C heteronuclear single quantum correlation (HSQC) NMR:** HSQC spectra were acquired on a Bruker NMR spectrometer (600 MHz) with the following parameters: 11 ppm sweep width in the F2 domain (¹H), 220 ppm sweep width in the F1 domain (¹³C), 8 scans, 512 increments, and a total acquisition time of approximately 1 h. Sample preparation involved the dissolution of a sample in (d₆-DMSO, Sigma-Aldrich, 99.5 atom % D). The evaluation was done according to [2].

**¹³C NMR:** ¹³C NMR spectra were recorded on a Bruker NMR spectrometer (600 MHz) using a 90° pulse and an inverse-gated decoupling sequence with a relaxation delay of 10 s, sweep width of 225 ppm, and 1024 scans. Samples were prepared by dissolving about 100 mg of product in deuterated chloroform (CDCl₃, Sigma-Aldrich, 99.5 atom% D).

**Gas chromatography - flame ionization detector (GC-FID):** First, a stock solution of model alkylphenols (2-isopropylphenol, CAS: 88-69-7, purity = 98%; phenol, CAS: 108-95-2, purity ≥ 99%; 4-methylphenol, CAS: 106-44-5, purity = 99%; 2,6-dimethoxyphenol, CAS: 91-10-1, purity = 99%; 2-methoxy-phenol, CAS: 90-05-1, purity ≥ 99%; 4-methylguaiacol, CAS: 93-51-6, purity ≥ 98%; 4-methyl-2,6-dimethoxyphenol, CAS: 6638-05-7, purity ≥ 97%; 4-ethylphenol, CAS: 123-07-9, purity = 99%; 4-ethyl-2-methoxy-phenol, CAS: 2785-89-9, purity ≥ 98%; 4-propylphenol, CAS: 645-56-7, purity = 99%; 2-methoxy-4-propylphenol, CAS: 2785-87-7, purity ≥ 99%; 2-methoxy-4-(2-propenyl)-phenol, CAS: 97-53-0, purity = 99%; 2,6-dimethoxy-4-prop-2-enylphenol, CAS: 6627-88-9, purity ≥ 95%; 4-(3-hydroxyprop-1-en-1-yl)-2-methoxyphenol, CAS: 458-35-5, purity = 98%; 4-(3-hydroxyprop-1-en-1-yl)-2,6-dimethoxyphenol, CAS: 537-33-7, purity = 80%, technical grade; 2-Methoxy-4-propenylphenol, CAS: 97-54-1, purity = 98%; 4-(3-Hydroxypropyl)-2-methoxyphenol, CAS: 2305-13-7, purity = 97%) in acetonitrile was prepared containing 10 mg/mL of each model compound. Other alkylphenol species were previously identified based on their retention time and mass spectra (MS). The stock solution was diluted with acetonitrile to obtain calibration solutions with concentrations of 500, 250, 125, 75, 50, 25, 10, and 5 µg/mL. Then, 50 mg of dry sample material was dissolved in 2 mL of THF at RT. 200 µL of acetonitrile, 200 µL of the calibration solutions, and 200 µL of the sample solution were transferred into separate GC-vials of 1.5 mL. 50 µL of N-methyl-N-(trimethylsilyl)trifluoroacetamide (MSTFA, CAS: 24589-78-4, purity ≥ 98.5%) and 650 µL of pyridine were added to each vial. The vials were closed and kept at 80 °C for 20 min. For the control of retention time, 100 µL of the sample solutions were transferred into additional 1.5 mL GC-vials and 100 µL of the 250 µg/mL-standard solution were added to each sample. 50 µL of MSTFA and 650 µL of pyridine were added, the vials were closed, and kept at 80 °C for 20 min. An aliquot of 1 µL of each vial was injected (injection T = 300 °C) into the GC-FID system (Trace 1600 Gas Chromatograph, Thermo Scientific Inc, USA). For the analysis, a DB-5ms column (30 m x 0.25 mm x 0.25 µm, Agilent Technologies Inc., USA) was used with He as a carrier gas at a flow rate of 0.7 mL/min. The oven was kept at 50 °C for 1 min and heated to 150 °C at 15 °C/min. 150 °C were kept for 6 min before further heating up to 305 °C at 10 °C/min. The oven was kept at 305 °C for 10 min. The total run time was 40 min. The FID temperature was 310 °C.

### Results:

### Dissolution of lignin:

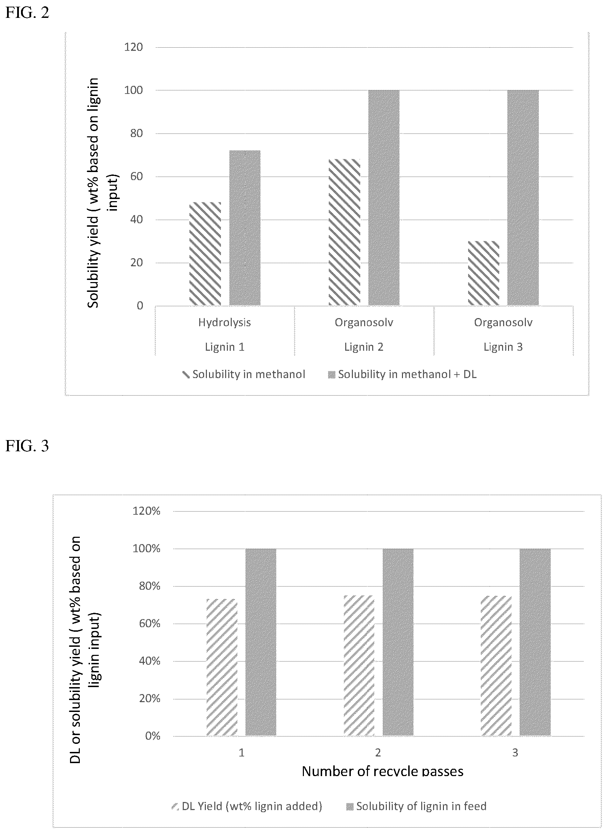

The dissolution of three different lignins in a solvent mixture of methanol and a (first) depolymerized lignin (DL1) was measured at temperatures ranging from 50 to 120°C. The optimal temperature for maximum dissolution yield varied depending on the lignin type and their solubility at optimal conditions is shown in Figure 2.

The two organosolv lignins tested had limited solubility in methanol alone (63 wt% and 33 wt%, respectively at 120 °C) but became completely soluble when DL1 was added at the same dissolution temperature. A similar trend was observed for hydrolysis lignin; however, its dissolution did not reach 100%, increasing only from 48 wt% in methanol alone to 76 wt% in the methanol-DL1 mixture.

The extraction method used to isolate the lignins is hypothesized to influence their solubility. Enzymes and acids used in hydrolysis-based fractionation processes often produce lignins that retain significant amounts of entrained sugars and heteroatom-containing compounds (e.g., enzyme-derived amino acids), which severely limit solubility in conventional organic solvents. Nevertheless, the use of DL as a solvent improved the overall solubility and pumpability of lignin.

### Overall liquid yields

Catalytic reductive depolymerization of organosolv lignin was carried out in a 100 mL batch reactor at 235°C with an initial pressure of 30 bar (at 25°C). The solvent system consisted of methanol and a Premixed Lignin (PL) (Lignin-DL mixture). In the start-up a first depolymerized lignin (DL1) was added in the PL. After this start-up, part of the depolymerized lignin obtained from the depolymerization step was recycled and added as the so named second depolymerized lignin (DL2) in the PL. Each of said cycles wherein DL2 is added in the PL, corresponds to the so named (recycling) passes in the present results. Figure 3 illustrates the solubility of lignin and the corresponding DL yield across different recycling passes (passes 1 - 3).

Solubility and the overall DL yield remained unchanged over the three recycle passes. Recycling the DL obtained for solubilization of fresh lignin showed no negative effect on the solubility of lignin (quantitative in all recycle passes 1-3). The DL yield similarly was unchanged during the experiments where obtained DL2 was recycled and ranged approximately around 78 - 78 wt%, on basis of lignin intake. After three recycling passes, the unchanged lignin solubility and DL yields demonstrate the robustness of the process. Char and gas formation were negligible across all passes. Additionally, since a significant fraction of ether linkages were cleaved during reductive catalytic depolymerization, a noticeable amount of water and lighter volatile organic compounds possibly originating from side chains in the lignin structure were released. However, this fraction was removed during work-up and was not reflected in the reported DL yield.

### Product analysis:

The depolymerized lignin (DL) obtained after reductive catalytic depolymerization of lignin was analyzed using various analytical techniques: GC-MS/FID for compositional analysis, GPC for molecular weight distribution, and 31P-NMR for assessing hydroxyl functionality.

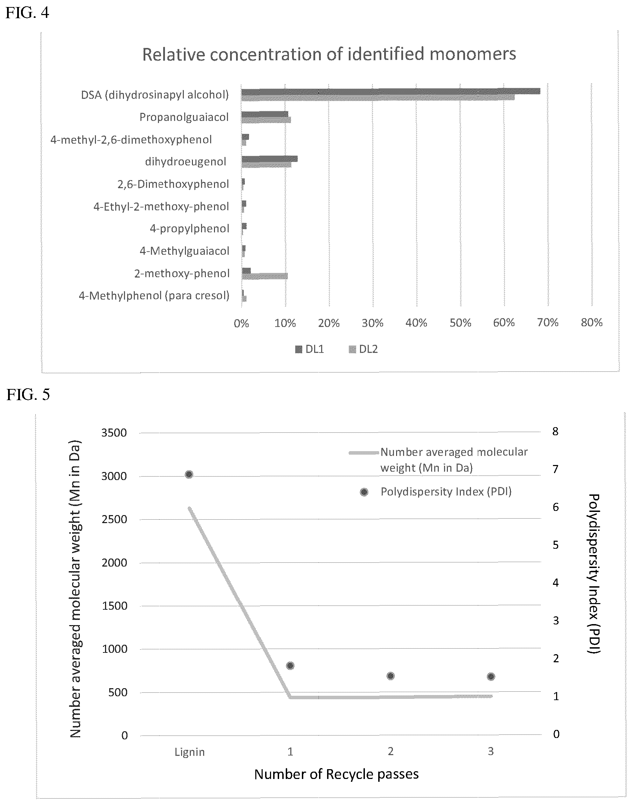

The compositions of two DL2 samples, produced from different recycling experiments (pass 1 and 3 respectively), were compared in Figure 4. These correspond to the DL2 samples from the recycling experiments discussed in Figure 2. The total monomer yield, quantified using a calibrated library of known alkylphenols, remained nearly identical at 22.33 wt% (Pass 1) and 23.28 wt% (Pass 4). Among the identified compounds, Depolymerized Lignin primarily consisted of dihydrosinapyl alcohol (DSA), which accounted for approximately 68 wt% of the GC-identifiable compounds. Apart from a slight increase in guaiacol concentration, no significant changes were observed in the alkylphenol composition of the DL2 due to recycling.

The molecular weight distribution of the DL2's obtained after different recycle passes were measured using GPC analysis. The number averaged molecular weight and the polydispersity index (PDI) of the DL2's obtained after different recycle passes in comparison to the polymeric lignin used as feedstock is shown in Figure 5. The average molecular weight and the PDI both drop significantly after depolymerization, from about 2600 Da and 7 to 55 Da and 1.2 respectively, signifying the extent of depolymerization that the lignin undergoes during RCD. Both the average molecular weight and the PDI remain approximately the same during the recycle runs where DL2 is recycled back in combination with methanol to completely dissolve the lignin.

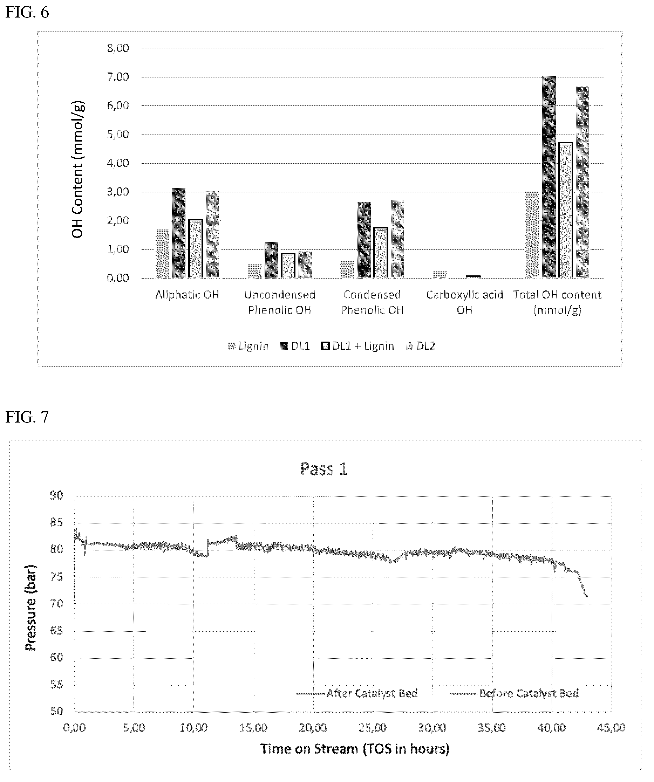

Key molecular changes, particularly in hydroxyl (OH) functionality due to the reductive catalytic depolymerization (RCD) process, were analyzed using 31P -NMR. The measured OH content as a function of DL recycling is shown in Figure 6, alongside the OH content of the lignin feedstock for comparison. The total OH content of the depolymerized lignin oil (7 mmol/g) increased significantly compared to the lignin feedstock (3 mmol/g). A noticeable increase in all OH functionalities was observed during depolymerization, except for the OH content associated with carboxylic acids, which decreased due to hydrogenation and hydrodeoxygenation reactions during RCD.

After four recycling passes, the OH content from different functionalities and the total OH content of the DL2 remained relatively unchanged, indicating that the recycling process did not degrade its quality.

### Continuous Depolymerization Experiments:

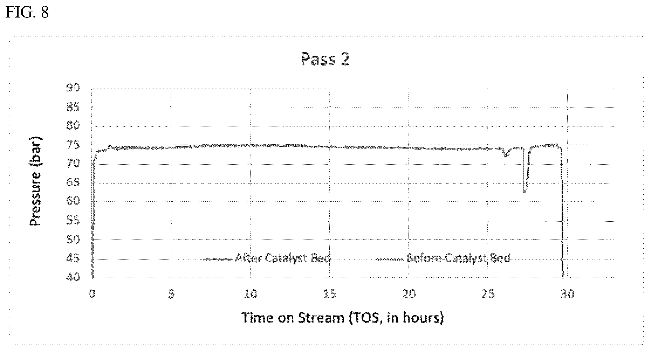

The reductive catalytic depolymerization of lignin was successfully demonstrated on a continuous scale over 80 hours of uninterrupted operation using a packed-bed reactor. The process was carried out at 230°C and 80 bar, with a hydrogen flow rate of 2000 NmL/h and heterogenous palladium catalyst. Two experimental runs were conducted: the first using pure methanol as solvent and the second utilizing a mixture of methanol and depolymerized lignin (DL1) from the initial experiment. The lignin exhibited a solubility of 33 wt% in methanol alone and 100% in the methanol-DL1 mixture, consistent with results from batch-scale experiments. Pressure profiles across the reactor bed for both experiments are shown in Figures 7 and 8. The absence of pressure fluctuations throughout both runs indicates that lignin condensation and char formation did not occur, confirming the feasibility of continuous operation. The yields and characteristics of the depolymerized lignin were comparable to those obtained from batch depolymerization experiments.

## Claims

1. A process for reductive catalytic depolymerization of lignin, the process comprising the steps of:
a) premixing a lignin (L) to be depolymerized with a first (in particular liquid) depolymerized lignin (DL1) in a weight ratio of DL1/L of at least 0.5, thereby obtaining a Premixed Lignin (PL);
b) liquifying the PL by adding an organic alcohol and heating with optionally pressurizing the thus obtained mixture at a temperature and pressure at which the organic alcohol is liquid, for a time sufficient to liquify the PL, thereby obtaining a liquified PL;
c) depolymerizing the liquified PL using reductive catalytic depolymerization, thereby obtaining a Product Mixture (PM) of the organic alcohol and depolymerized lignin; and
d) removing the organic alcohol from the PM, thereby obtaining a second (in particular liquid) depolymerized lignin (DL2).

2. The process according to claim 1, further comprising recycling a fraction of the second depolymerized lignin (DL2) obtained in step d) to step a).

3. The process according to claim 1 or 2, wherein step b) comprises pressurizing the liquified PL to a pressure up to 100 bar prior to the depolymerizing step.

4. The process according to any one of claims 1 to 3, further comprising a separation step wherein solids are removed from the liquified PL prior to the depolymerization step c); in particular using a filtration system.

5. The process according to any one of claims 1 to 4, wherein the reductive catalytic depolymerization is performed at a temperature ranging from 200°C to 270°C; in particular at pressures ranging from 10 to 100 bars.

6. The process according to any one of claims 1 to 5, wherein the reductive catalytic depolymerization is performed using a heterogeneous catalyst, typically composed of noble metals such as Pd/Ru/Pt/Rh and the like or transition metal catalysts such as Ni/Co/Cu.

7. The process according to any one of claims 1 to 6, wherein the organic alcohol is selected from the group consisting of methanol, ethanol, propanol, butanol, and mixtures thereof.

8. The process according to any one of claims 1 to 7, wherein the heating in step b) is performed at temperatures up to 150°C; in particular at temperatures ranging from 50°C to 150°C, preferably for a time period ranging from 5 minutes to 1 hour.

9. The process according to any one of claims 1 to 8, wherein the weight ratio of DL/L ranges from 0.5 to 5; in particular from 0.5 to 2.

10. The process according to any one of claims 1 to 9, wherein the lignin (L) is selected from the group consisting of kraft lignin, organosolv lignin, soda lignin, and lignosulfonate.

11. The process according to any one of claims 1 to 10, wherein the reductive catalytic depolymerization is performed in a continuous mode using a packed bed reactor or in batch mode using a pressured autoclave reactor; preferably in a continuous mode using a packed bed reactor.

12. The process according to any one of claims 1 to 11, wherein in step d) the organic alcohol is removed from the depolymerized lignin through a flash step or by distillation under reduced pressure.

13. The process according to any one of claims 1 to 12, wherein the premixing of step a) is done using a screw extruder system.

14. The process according to any one of claims 1 to 13, wherein the process is a continuous process.

15. The process according to any one of claims 1 to 14, wherein the depolymerization step results in minimal losses of carbon as undissolved lignin or char.

16. The process according to any one of claims 1 to 15, wherein the DL2 in step d) is substantially identical in composition to the DL1 in step a) .

17. A liquid depolymerized lignin obtained by the process according to any one of claims 1 to 16.
